# EUROPEAN PATENT APPLICATION

(11) **EP 1 810 668 A1**
(43) Date of publication of application: **25.07.2007**
(21) Application number: 06001314.1
(22) Date of filing: 23.01.2006
(51) Int. Cl.: A61K 9/16, A61K 9/50, A61K 31/137

(54) **Coated formulations containing tolterodine tartrate**

(71) Applicant: LEK Pharmaceuticals d.d., 1526 Ljubljana (SI)
(72) Inventor: Legen, Igor, 1290 Grosuplje (SI); Kuhar, Polonca, 1000 Ljubljana (SI)
(74) Representative: Dietz, Jörg-Reimar

(57) **Abstract**

A pharmaceutical composition of a drug which may have increased plasma concentrations due to the concomitant use of antacids has been developed which comprises coating with the pH-dependent dissolution, characterized by the rapid dissolution at acidic conditions (pH < 5.5) and very slow dissolution at higher pH values

## Description

### FIELD OF THE INVENTION

The present invention relates to the field of pharmaceutics, and specifically to pharmaceutical formulation comprising a coating exhibiting a pH-dependent dissolution, characterized by the rapid dissolution at acidic conditions (pH < 5.5, preferably < 5, more preferably at pH in stomach) and very slow dissolution at elevated (meaning more alkaline than 5,5, preferably 6) pH values.

### BACKGROUND OF THE INVENTION

Many active pharmaceutical ingredients (drugs thereon) exhibit pH-dependent dissolution and/or pH-dependent stability. For these drugs and formulations thereof the change of the gastric pH can significantly affect the release of the drug from the formulation, which results in the altered pharmacokinetics and consequently altered pharmacodynamics of the drug. In the case of the increased plasma concentration of the drug the patient may experience enhanced side effects, while decreased plasma concentrations could result in the lack of the efficacy of the drug.

One of the most frequent change of the gastric pH is the elevation of the gastric pH caused by antacids. Antacids, including specifically group consisting of: magnesium carbonate, calcium carbonate, sodium bicarbonate, magnesium hydroxide, magnesium oxide, aluminum carbonate, aluminum hydroxide, aluminum phosphate, magnesium trisilicate, megaldrate, almagate, dihydroxyaluminum aminoacetate, and dihydroxyaluminum sodium carbonate work by neutralizing the excess of the stomach acid. For example, it was demonstrated that orally taken almagate (magnesium carbonate-aluminium hydroxide) increased gastric pH from about 1.2 to about 6.0 in about 5.5 minutes (Aliment. Pharmacol. Ther., 20, 683-688, 2004). Antacids are widely used for the relief of the gastro-oesopahageal reflux symptoms which show a high prevalence in general population (30-60%), antacids are also used for the treatment of duodenal ulcers, gastric ulcers, stress gastritis, bile acid mediated diarrhea, biliary reflux, constipation (Drugs, 57, 855-870, 1999) thus one can expect frequent concomitant use of antacids with other medications, including drugs and pharmaceutical formulations with pH-dependent dissolution and or pH-dependent stability.

A condition that is very frequently connected to the concomitant use of antacids with other medications is for example stress. It is noted that stress contributes to the etiology of between 30 % and 65 % of peptic ulcer cases (Psychosom. Med., 62, 176-185, 2000), and antacids are commonly used for the treatment of upper gastrointestinal ulcers (Drugs, 57, 855-870, 1999). It is also noted that stress is related to several diseases, including urinary incontinence (Int. J. Gynaecol. Obstet., 82, 327-338, 2003), heart and coronary diseases (Curr. Opin. Cardio.I, 19, 494-499, 2004), hypertension (Metabolism, 51, 40-45, 2002), Alzheimer's disease, Parkinson's disease, cancer, chronic renal disease, chronic locomotor system diseases, chronic intestinal diseases, chronic liver disease, gallbladder disease, polycystic ovary syndrome, prostatic hyperplasia, type 2 diabetes, infections (Clin. Nutr, 23, 1256-1266, 2004).

Drugs with the decreased plasma concentrations defined by the decreased peak plasma concentration (C_{MAX}) and/or area under the curve (AUC) due to increase of the gastric pH and consequently impaired *in vivo* dissolution are for example poorly-water soluble weakly basic drugs. Specifically: antifungal agent ketoconazole, anti-emetic agent cinnarizine, antibacterial agents enoxacin and cefpodoxime proxetil, antianxiety agent diazepam. Additionally, calcium salts and zinc salts also exhibit impaired *in vivo* dissolution due to elevated gastric pH. On the other hand a drug with the increased plasma concentrations defined by the increased C_{MAX} due to the concomitant use of antacids and consequently the increased *in vivo* dissolution of the drug is for example an urinary antispasmodic agent tolterodine in its tartrate salt form, thereon term tolterodin will comprise tolterodin or any salt or metabolite thereof, preferably tolterodine tartrate.

Elevated gastric pH may also afford the enhanced absorption of the acid-labile drugs, which may be preferably: penicillins, erythromycin, used as antiinfectives or digoxin used to treat congestive heart failure or proton pump inhibitors.

Elevated gastric pH might also change the pharmacokinetic properties of the coated formulations (i.e. enteric coated ketoprofen tablets), because the pH in the stomach increase as a result of the concomitant use of the antacids to the values that are characteristic for the small intestine, thus the release of the drug may begin already in the stomach, resulting in shorter T_{MAX} (time to reach maximum concentration) and increased C_{MAX}.

The high probability of impaired gastric pH associated with diseases and conditions selected form the above-mentioned groups, particularly urinary diseases thus requires that drugs used for the manufacturing of the medicament for treatment of above diseases and/or conditions are formulated into a pharmaceutical composition exhibiting predictable pH dependant dissolution characteristics; especially if they are to be used concomitantly.

### DISCLOSURE OF THE INVENTION

The present invention is in a broad sense a coated pharmaceutical composition, characterized by the rapid dissolution at acidic conditions (pH < 5.5) and slow dissolution at higher pH values, in particular it will comprise an active pharmaceutical ingredient which has increased plasma concentrations if administered concomitantly with an antacid, more particularly the active pharmaceutical ingredient is tolterodin.

In a specific aspect the invention is a pharmaceutical composition comprising tolterodin tartrate, said composition having a coating with the pH-dependent dissolution, characterized by the rapid dissolution at pH < 5.5 and slow dissolution at higher pH values, in particular the coating comprises a polymer or copolymer of acrylate and/or methacrylate with quarternary ammonium group, such as dimethylaminoethyl methacrylate (Eudragit E PO).

In one embodiment of the invention the pharmaceutical composition the rapid dissolution at pH < 5.5 is defined by that at pH 2.0 the coating is completely dissolved in less than 30 minutes if subjected to the dissolution test in apparatus 2 in accordance with USP 28 and/or the slow dissolution at pH values above 5.5 is defined by that at pH 6.8 the coating is substantially not dissolved after 180 minutes if subjected to the dissolution test in apparatus 2 in accordance with USP 28. Specifically this is characterized in that that in the solution or the suspension less than 20% by weight of the amount of polymer used in coating is detected, preferably by HPLC, after 3 h, when subjecting the composition to dissolution test in apparatus 2 in accordance with USP 28.

In an alternative embodiment the dissolution characteristics of said composition are characterized in that that less than 20% by weight of active pharmaceutical ingredient is released at pH = 6,8 within 3 h if subjected to the dissolution test in apparatus 2 in accordance with USP 28.

The pharmaceutical composition of our invention in an aspect consists of cores coated with a first coating wherein cores comprise an active pharmaceutical ingredient, and a second separating coating and a third coating comprising copolymer of acrylate and methacrylate with quarternary ammonium group, preferably wherein the second coating comprises hydrocypropyl cellulose, more preferably where the active pharmaceutical ingredient is tolterodin, more preferably where the coated cores coated are pellets, which are filled into capsule or compressed into tablets.

In a specific aspect the invention is a pharmaceutical composition, characterized in that it consists of pellet which comprise tolterodine tartarte which are coated by one or optionally more coatings and at least one of said coatings comprises a polymer or copolymer of acrylate and/or methacrylate with quarternary ammonium group, preferably Eudragit E PO or Eudragit E 100.

In yet more specific aspect the invention is a pharmaceutical composition, characterized in that it consists of pellet cores comprising tolterodine tartarte coated with a first coating, a second coating and a third coating comprising Eudragit E PO, sodium lauryl sulfate, stearic acid, and magnesium stearate, preferably where second coating comprises ethylcellulose, and hydroxypropyl cellulose.

Use of the coating characterized by the pH-dependent dissolution which is rapid at acidic conditions (pH < 5.5) and very slow at higher pH values for neutralizing the effect of the elevated pH on the release and/or stability of the drug and/or formulations that exhibit pH-dependent dissolution and/or stability, caused by the concomitant use of the antacids is contemplated within the scope of the invention, in particular the use of a copolymer of acrylate and methacrylate with quarternary ammonium group for coating a pharmaceutical composition comprising tolterodin.

The invention is for example embodied in a process for manufacturing a pharmaceutical composition characterized in that it consists of following steps:
a) preparing cores comprising an active pharmaceutical ingredient;
b) applying a coating to said cores, characterized in that the coating layer provides for the rapid dissolution at pH < 5.5 and slow dissolution at higher pH values. In particular the coating applied in step b) comprises aminoylkyl methacrylate copolymers and/or chitosan or chitosan derivatives.

More specifically the invention is embodied in a process for manufacturing a pharmaceutical composition characterized in that it consists of following steps:
a) Preparing cores comprising an active pharmaceutical ingredient;
b) applying an enteric coating or release controlling agent to said cores;
c) applying second separating coating to said first coated cores, giving second cores;
d) applying third coating to said second cores, characterized in that third coating layer provides for the rapid dissolution at pH < 5.5 and slow dissolution at higher pH values. In particular the coating applied in step d) comprises a polymer selected from aminoalkyl methacrylate copolymers, (preferably a copolymer of acrylate and methacrylate with quarternary ammonium group), chitosan or chitosan derivatives, in particular also the coating in step c) comprises a cellulose derivative such as and preferably hydroxypropyl cellulose.

The aspect of the invention are also the use of the above described composition for treatment of patient who is being concomitantly treated with antacid.

In summary the general aspect of the invention is thus the use of coating with the pH-dependent dissolution, characterized by the rapid dissolution at acidic conditions (pH < 5.5) and very slow dissolution at higher pH values for manufacturing a medicament; in particular wherein the medicament comprises tolterodin and the use of so coated compositions for manufacturing a medicament for treating an urinary difficulty, which is preferably a frequent urination and/and inability to control urination

The method of treatment aspects of the invention are the treating urinary diseases by administering above described compositions in particular treating urinary difficulty.

Yet another aspect of the invention is a method of administering urinary antispasmodic medicament, by using a described composition wherein the administration is preformed concomitantly with administration of an antacid.

### DETAILED DESCRIPTION OF THE INVENTION

The present invention describes the use of the coating in the manufacturing of a pharmaceutical composition with the pH-dependent dissolution, characterized by the rapid dissolution thereof at acidic conditions, that is at pH < 5.5; which means that at pH 2.0 the coating comprising a coating agent is completely dissolved in less than 30 minutes, preferably in less than 20 minutes, more preferably in less than 15 minutes (if coating dissolution is measured under sink conditions in an USP 28 dissolution apparatus 2) and slow dissolution at pH values higher than 5.5 that is at pH 6.8 the coating is substantially not dissolved even after 180 minutes (if coating dissolution is measured under sink conditions in an USP 28 dissolution apparatus 2). The fact that coating is substantially not dissolved may be characterized in one embodiment by measuring the release active pharmaceutical ingredient (API) wherein the release of API is not a limited or modified factor, in that at pH = 6,8 (0.1 M phosphate buffer, USP 28 apparatus 2) less than 30% of API is released within 3 h, preferably less than 20% after 1 h; more preferably less than 10% after 2 h. In the other embodiment it may be characterized by the fact that in the solution or the suspension (when subjecting the coated composition to dissolution test in apparatus 2 in accordance with USP 28) less than 20% by weight of the amount of polymer (coating agent) used in coating is detected after 3 h, preferably less than 10% after 1 h, more preferably less than 10% after 2 h, still more preferably less than 10 after 3 h; which can be conveniently measured spectrofotometricaly or alternatively by HPLC or MS or other analytical technique.

This coating is intended to neutralize the effect of the elevated pH on the release/stability of the drugs and formulations that exhibit pH-dependent dissolution and/or stability, in particular caused by the concomitant use of the antacids and is applied in the pharmaceutical composition in a manner that it comes in the contact with the gastric content sooner then the drug. It is contemplated that the coating primarily influences the pH dependence of the dissolution and/or stability characteristics of the drugs and/or pharmaceutical compositions, however the cores being coated may be also formulated (manufactured) in a way to provide modified release after the coating has dissolved or disintegrated.

Coating preferably contains a polymer or combination of polymers which contain alkaline functional groups. Polymers are selected from the purely synthetic materials such as aminoalkyl methacrylate copolymers or from the natural polymers such as chitosan and chitosan derivatives. Under the normal acidic conditions in the stomach this coating is rapidly dissolved without affecting the drug release from the pharmaceutical formulation. If antacids are concomitantly used with the drugs and pharmaceutical formulation that exhibit pH-dependent dissolution and/or stability the above mentioned coating prevents the release of the drug from the pharmaceutical formulation and thus neutralize the effect of elevated pH on the drug release/stability. When pH in the stomach falls under the pH at which the above mentioned coating is dissolved (i.e. in the case of orally taken almagate the gastric pH, after being increased to about 6.0 in about 5.5 minutes, decreased under 5.5 in about 10 minutes (Aliment. Pharmacol. Ther., 20, 683-688, 2004)) as a result of the ceasing of the antacid activity, the coating is dissolved and the drug or pharmaceutical formulation is exposed to more normal acidic conditions in the stomach, enabling the more predictive pharmacokinetic and consequently more predictive efficacy of the drug.

### Formulation to avoid enhanced absorption of drugs

In the case of the acid-labile drugs elevated gastric pH may result in enhanced absorption and subsequently enhanced adverse effects. This undesired effect can be circumvented by coating the cores, which may be either crystals, granules, pellets, tablets or capsules comprising above mentioned drug and suitable, pharmaceutical excipients, with a coating layer of a coating agent, preferably an anionic polymer containing alkaline functional groups, more preferably an acrylate polymer, most preferably copolymer of acrylate and methacrylates with quarternary ammonium group, such as Eudragit E PO. The following table shows a typical weight (relative to finished composition) composition of a coating to be applied to the cores of the pharmaceutical composition, whereas the coating amounts to 10 - 20 % by weight to the finished composition.

| | | |
|---|---|---|
| coating agent | polymer (preferably: Eudragit E PO) | 6,0-12,0 |
| surfactant | sodium lauryl sulfate, polysorbate 80 | 0,5-1,5 |
| plasticizer | stearic acid, dibutyl sebacate, acetyltributyl citrate | 0,5-1,5 |
| glidant | magnesium stearate, talc | 2,0-5,0 |

Cores, preferably pellets are prepared, preferably by extrusion and spheronisation of wet mass consisting of API, sufficient amount of spheronising agent (i. e. microcrystalline cellulose), optionally release controlling agent and granulation fluid (i.e. water, ethanol). Dry cores are coated with the coating dispersion prepared in following steps. First, surfactant, preferably a sodium lauryl sulfate, is dissolved in purified water and plasticizer. preferably containing stearic acid and/or dibutyl sebacate and/or acetyltributyl citrate or similar excipient, preferably stearic acid, added while stirring (e.g. by propeller mixer). Then, coating agent is dispersed into prepared liquid and mixed (e.g. with high shear mixer) until clear, yellowish dispersion is formed. Separately, suspension of glidant in water is prepared, added to polymer dispersion and mixed. The resulting dispersion is used for coating of the cores in a fluid-bed device.

### Formulation to avoid premature release of coated composition

Due to elevated gastric fluid pH changes in the pharmacokinetic properties of coated , preferably enteric coated, formulations may occur and the release of the drug may begin already in the stomach. Additional coating layer comprising of polymer with cationic properties is able to prevent this undesired effect. Due to possible interactions between anionic polymer for first coating and cationic polymer for protective coating additional inert separating coating layer may be required. First coated material may be in the form of coated crystals, granules, pellets, tablets or capsules.

The following table shows a typical weight (relative to finished composition) composition of a separating (second) coating to be applied to the preferably enteric coated cores of the pharmaceutical composition, whereas the coating amounts to 3 -30 % by weight to the finished composition. Composition will depend on the first and third coating.

| | | |
|---|---|---|
| coating agent | hydroxypropyl cellulose | preferably 2-5 |
| plasticizer | polyethylene glycol | preferably 0,2-0,5 |
| glidant | talc | preferably 0,5-1,0 |

Bellow is a typical weight (relative to finished composition) composition of a (third) coating to be applied to the cores of the pharmaceutical composition, whereas the coating amounts to 10 - 20 % by weight to the finished composition.

| | | |
|---|---|---|
| coating agent | polymer (preferably: Eudragit E PO) | 6,0-12,0 |
| surfactant | sodium lauryl sulfate | 0,5-1,5 |
| plasticizer | stearic acid | 0,5-1,5 |
| glidant | magnesium stearate | 2,0-5,0 |

Cores, preferably pellets are prepared by extrusion and spheronisation of wet mass consisting of active substance, sufficient amount of spheronising agent (e.g. microcrystalline cellulose), optionally release controlling agent and granulation fluid (i.e. water, ethanol).
Dry cores are first coated with separating layer, prepared in following steps: (a) coating agent, preferably powdered hydroxypropyl cellulose, but also hydroxypropylmethylcellulose or methylcellulose is slowly added to well-agitated liquid, preferably water at room temperature and stirred until a gel-free solution is obtained, (b) plasticizer, preferably polyethylene glycol, but also glycerol, propylene glycol, triacetin is stirred into solution, (c) glidant, preferably talc suspension in suitable liquid, preferably water, is prepared separately (e.g. with high-shear mixing) and (d) combined with above solution. Cores are coated and preferably dried in a fluid bed device.
Finally, the protective coating is applied on coated cores. The coating dispersion is prepared in following steps: (a) surfactant, preferably sodium lauryl sulphate but also a poloxamer, is dissolved (e.g. in purified water) and a plasticizer, preferably stearic acid, is added while stirring (e.g. with propeller mixer), (b) coating agent (a polymer) is dispersed into prepared liquid and mixed (e.g. with high shear mixer) until clear dispersion is formed Separately, suspension of glidant, preferably magnesium stearate or also talc or glycerol monostearat, (e.g. in water) is prepared (c), added to above dispersion and mixed. The resulting dispersion (d) is used for coating of the cores in a fluid-bed device.

Working Example 1

| **Pellet cores (weight per capsule =160 mg)** | |
|---|---|
| Tolterodine tartarte | 4.00 mg |
| Microcrystalline cellulose | 156.00 mg |
| Purified water | 180.00 mg |

| **Coating (15 % application, based on coated pellets weight)** | |
|---|---|
| Eudragit E PO | 15,00 mg |
| Sodium lauryl sulfate | 1,50 mg |
| Stearic acid | 2,00 mg |
| Magnesium stearate | 6,00 mg |
| Purified water | 140,0 mg |

Tolterodine tartrate (API) and microcrystalline cellulose are mixed and granulated with water. Granulate is coated by a dispersion prepared as follows: sodium lauryl sulphate is dissolved in purified water and stearic acid is added while stirring, thereto a polymer is dispersed and mixed dispersion is formed. Thereto separately prepared suspension of magnesium stearate in water is added to polymer and the resulting dispersion is used for coating.

### Working Example 2

| **Pellet cores (weight per capsule = 160 mg)** | |
|---|---|
| Tolterodine tartarte | 4.000 mg |
| Microcrystalline cellulose | 156.000 mg |
| Purified water | 180.000 mg |

| **Separating coating (15 % application based on core weight)** | |
|---|---|
| Ethylcellulose | 26,253 mg |
| Hypromellose | 2,172 mg |
| Purified water | 110,769 mg |

| **Coating (10 % application, based on coated pellets weight)** | |
|---|---|
| Eudragit E PO | 13,07 mg |
| Sodium lauryl sulfate | 1,31 mg |
| Stearic acid | 1,96 mg |
| Magnesium stearate | 4,57 mg |
| Purified water | 118,52 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. First, ethylcellulose polymer dispersions with purified water is prepared. Separately, hypromellose is dissolved in water, added to ethylcellulose dispersion and mixed. The resulting dispersion is used for coating of the pellet cores in a fluid-bed device. Subsequnetly the protective coating is applied on dry coated pellets. The coating dispersion is prepared in following steps: (a) sodium lauryl sulphate is dissolved in purified water and stearic acid is added while stirring with propeller mixer, (b) polymer is dispersed into prepared liquid and mixed with high shear mixer until clear, yellowish dispersion is formed. Separately, suspension of magnesium stearate in water is prepared (c), added to polymer dispersion and mixed. The resulting dispersion (d) is used for coating of the pellet cores in a fluid-bed device.

### Working Example 3

| **Pellet cores (weight per capsule = 160 mg)** | |
|---|---|
| Tolterodine tartarte | 4.00 mg |
| Microcrystalline cellulose | 156.00 mg |
| Purified water | 180.00 mg |

| **Coating (20 % application, based on coated pellets weight)** | |
|---|---|
| Dimethylaminoethyl methacrylate (Eudragit E 100) | 32,40 mg |
| Polyethylene glycol 6000 | 2,00 mg |
| Talc | 7,60 mg |
| Isopropyl alcohol | 212,40 mg |
| Acetone | 141,60 mg |
| Purified water | 4,00 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. One part (2/3) of Isopropyl alcohol and acetone are introduced first and then Eudragit E 100 is added while stirring with a propeller stirrer for aprox. 30-60 minutes until a clear solution is formed. Separately, polyethylene glycol 6000 is dissolved in water and slowly added to remaining isopropyl alcohol. Then talc is added and suspension is homogenized for 20 minutes. Finally, the talc suspension is added to the polymer solution with gentle stirring.

The coating suspension is applied to the pellet cores in fluid bed device.

### Working Example 4

| **Pellet cores (weight per capsule =160 mg)** | |
|---|---|
| Tolterodine tartarte | 4.000 mg |
| Microcrystalline cellulose | 156.000 mg |
| Purified water | 180.000 mg |

| **Separating coating (15 % application based on core weight)** | |
|---|---|
| Ethylcellulose | 26,253 mg |
| Hypromellose | 2,172 mg |
| Purified water | 110,769 mg |

| **Coating (20 % application, based on coated pellets weight)** | |
|---|---|
| Eudragit E PO | 29,41 mg |
| Sodium lauryl sulfate | 2,94 mg |
| Stearic acid | 4,42 mg |
| Magnesium stearate | 10,33 mg |
| Purified water | 247,01 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. First, ethylcellulose polymer dispersions with purified water is prepared. Separately, hypromellose is dissolved in water, added to ethylcellulose dispersion and mixed. The resulting dispersion is used for coating of the pellet cores in a fluid-bed device. Subsequnetly the protective coating is applied on dry coated pellets. The coating dispersion is prepared in following steps: (a) sodium lauryl sulphate is dissolved in purified water and stearic acid is added while stirring with propeller mixer, (b) polymer is dispersed into prepared liquid and mixed with high shear mixer until clear, yellowish dispersion is formed. Separately, suspension of magnesium stearate in water is prepared (c), added to polymer dispersion and mixed. The resulting dispersion (d) is used for coating of the pellet cores in a fluid-bed device.

## Claims

1. A coated pharmaceutical composition, **characterized by** the rapid dissolution at acidic conditions (pH < 5.5) and slow dissolution at higher pH values.

2. The pharmaceutical composition in accordance with the previous claim comprising an active pharmaceutical ingredient which has increased plasma concentrations if administered concomitantly with an antacid.

3. The pharmaceutical composition in accordance with the s claim 1 wherein the active pharmaceutical ingredient is tolterodin.

4. A pharmaceutical composition comprising tolterodin tartrate, said composition having a coating with the pH-dependent dissolution, **characterized by** the rapid dissolution at pH < 5.5 and slow dissolution at higher pH values.

5. The pharmaceutical composition in accordance with the previous claim wherein the coating comprises a polymer or copolymer of acrylate and/or methacrylate with quarternary ammonium group.

6. The pharmaceutical composition in accordance with the previous claim wherein the coating comprises a polymer or copolymer of dimethylaminoethyl methacrylate

7. The pharmaceutical composition in accordance with any of the previous claims wherein the rapid dissolution at pH < 5.5 is defined by that at pH 2.0 the coating is completely dissolved in less than 30 minutes if subjected to the dissolution test in apparatus 2 in accordance with USP 28.

8. The pharmaceutical composition in accordance with any of the previous claims wherein slow dissolution at pH values above 5.5 is defined by that at pH 6.8 the coating is substantially not dissolved after 180 minutes if subjected to the dissolution test in apparatus 2 in accordance with USP 28.

9. The pharmaceutical composition according to any of the previous claims **characterized in that that** less than 20% by weight of active pharmaceutical ingredient is released at pH = 6,8 within 3 h if subjected to the dissolution test in apparatus 2 in accordance with USP 28.

10. The pharmaceutical composition according to any of the previous claims **characterized in that** that in the solution or the suspension less than 20% by weight of the amount of polymer used in coating is detected after 3 h, when subjecting the composition to dissolution test in apparatus 2 in accordance with USP 28.

11. The pharmaceutical composition according to the previous claim, wherein the amount of polymer is detected by HPLC in solution or dispersion after subjecting the composition to dissolution test in apparatus 2 in accordance with USP 28.

12. The pharmaceutical composition according to any of the previous claims, **characterized in that** it consists of cores coated with a first coating wherein cores comprise an active pharmaceutical ingredient, and a second separating coating and a third coating comprising copolymer of acrylate and methacrylate with quarternary ammonium group.

13. The pharmaceutical composition according to previous claim wherein the second coating comprises hydrocypropyl cellulose.

14. The pharmaceutical composition in accordance with previous claim where the active pharmaceutical ingredient is tolterodin.

15. The pharmaceutical composition in accordance with previous claim where the coated cores coated are pellets, which are filled into capsule or compressed into tablets.

16. A pharmaceutical composition, **characterized in that** it consists of pellet which comprise tolterodine tartarte which are coated by one or optionally more coatings and at least one of said coatings comprises a polymer or copolymer of acrylate and/or methacrylate with quarternary ammonium group.

17. The pharmaceutical composition in accordance with previous claim wherein said a polymer or copolymer is Eudragit E PO or Eudragit E 100.

18. A pharmaceutical composition, **characterized in that** it consists of pellet cores comprising tolterodine tartarte coated with a first coating, a second coating and a third coating comprising Eudragit E PO, sodium lauryl sulfate, stearic acid, and magnesium stearate.

19. The pharmaceutical composition according to the previous claim **characterized in that** a second coating comprises ethylcellulose, and hydroxypropyl cellulose.

20. Use of the coating **characterized by** the pH-dependent dissolution which is rapid at acidic conditions (pH < 5.5) and very slow at higher pH values for neutralizing the effect of the elevated pH on the release and/or stability of the drug and/or formulations that exhibit pH-dependent dissolution and/or stability, caused by the concomitant use of the antacids.

21. Use of a copolymer of acrylate and methacrylate with quarternary ammonium group for coating a pharmaceutical composition comprising tolterodin.

22. A process for manufacturing a pharmaceutical composition **characterized in that** it consists of following steps:
a) preparing cores comprising an active pharmaceutical ingredient;
b) applying a coating to said cores, **characterized in that** the coating layer provides for the rapid dissolution at pH < 5.5 and slow dissolution at higher pH values.

23. A process according to previous claim, wherein active pharmaceutical ingredient is tolterodin and the coating applied in step b) comprises aminoylkyl methacrylate copolymers and/or chitosan or chitosan derivatives.

24. A process for manufacturing a pharmaceutical composition **characterized in that** it consists of following steps:
a) Preparing cores comprising an active pharmaceutical ingredient;
b) applying an enteric coating or release controlling agent to said cores;
c) applying second separating coating to said first coated cores, giving second cores;
d) applying third coating to said second cores, **characterized in that** third coating layer provides for the rapid dissolution at pH < 5.5 and slow dissolution at higher pH values.

25. A process in accordance with previous claim where the coating in step d) comprises a polymer selected from aminoalkyl methacrylate copolymers, chitosan or chitosan derivatives.

26. A process in accordance with previous claim wherein the aminoalkyl methacrylate copolymer is a copolymer of acrylate and methacrylate with quarternary ammonium group.

27. A process in accordance with any of the previous 3 claims where the coating in step c) comprises hydroxypropyl cellulose.

28. Use of a composition in accordance with any of claims 1 to 18 for treatment of patient who is being concomitantly treated with antacid.

29. Use of coating with the pH-dependent dissolution, **characterized by** the rapid dissolution at acidic conditions (pH < 5.5) and very slow dissolution at higher pH values for manufacturing a medicament.

30. Use in accordance with previous claim, wherein the medicament comprises tolterodin.

31. Use in accordance with previous claim for manufacturing a medicament for treating an urinary difficulty.

32. Use in accordance with previous claim where urinary difficulty is frequent urination and/and inability to control urination

33. Method of treating urinary diseases by administering a composition in accordance with any of claims 1 to 18.

34. Method in accordance with previous claim, wherein the urinary disease is an urinary difficulty.

35. Method in accordance with previous claim, wherein the urinary difficulty is frequent urination and/and inability to control urination

36. Method of administering urinary antispasmodic medicament by using a composition in accordance with any of claims 1 to 18 wherein the administration is preformed concomitantly with administration of an antacid.
